# EUROPÄISCHE PATENTSCHRIFT

(11) **EP 0 624 567 B1**
(45) Veröffentlichungstag und Bekanntmachung des Hinweises auf die Patenterteilung: **08.12.1999**
(21) Anmeldenummer: 94106897.5
(22) Anmeldetag: 03.05.1994
(51) Int. Cl.: C07C 215/76, C07C 235/46, C07C 217/86, C07C 255/54, C07C 217/46

(54) **Derivate des 3-Fluorphenols, Verfahren zu ihrer Herstellung und ihre Verwendung**
Derivatives of 3-fluorophenol, process for their preparation and their use
Dérivés de 3-fluorophénol, procédé de leur préparation et leur utilisation

(30) Priorität: 11.05.1993 DE 4315625
(43) Veröffentlichungstag der Anmeldung: 17.11.1994
(73) Patentinhaber: Clariant GmbH, 65929 Frankfurt am Main (DE)
(72) Erfinder: Pfirmann, Ralf Dr., D-64347 Griesheim (DE); Papenfuhs, Theodor Dr., D-60433 Frankfurt am Main (DE); Forstinger, Klaus Dr., D-65451 Kelsterbach (DE)

(56) Entgegenhaltungen:
- EP-A- 0 353 631
- EP-A- 0 375 076
- EP-A- 0 402 751
- EP-A- 0 462 800
- GB-A- 2 190 376
- CHEMICAL ABSTRACTS, vol. 116, no. 19, 1992, Columbus, Ohio, US; abstract no. 194304j, & CN-A-1 053 921 (BOOTS CO. PLC) 21. August 1991
- CHEMICAL ABSTRACTS, vol. 115, no. 25, 1991, Columbus, Ohio, US; abstract no. 279943w, & J. HETEROCYCL. CHEM., Bd.28, Nr.5, 1991 Seiten 1357 - 1363 J.B. HYNES ET AL
- CHEMICAL ABSTRACTS, vol. 110, no. 15, 1989, Columbus, Ohio, US; abstract no. 135186a, & J. HETEROCYCL. CHEM., Bd.25, Nr.4, 1988 Seiten 1173 - 1177 J.B.HYNES ET AL
- N.H.P. CNUBBEN ET AL: "Study on the regioselectivity and mechanism of the aromatic hydroxylation of monofluoroanilines", CHEM.-BIOL. INTERACTIONS, Ireland, Band 85, Seiten 151 - 172
- N. HAGA ET AL: "Acid-catalized amino-migration of O-phenylhydroxylamines", J. AM. CHEM. SOC., Band 114, Seiten 9795 - 9806

## Beschreibung

Die vorliegende Erfindung betrifft neue Derivate des 3-Fluorphenols, Verfahren zu ihrer Herstellung und ihre Verwendung.

Die EP-A-0 353 631 betrifft Isoxazolbenzoxazepine, ein Verfahren zu ihrer Herstellung und ihre Verwendung als Medikamente. In Beispiel 1 (b) ist die Herstellung von 2-Fluor-6-methoxymethoxybenzonitril beschrieben.

Im Zusammenhang mit der Herstellung von Benzopyranpyrazolonen nennt Chemical Abstracts Vol. 116, Nr 19, 194304j (1992) und Seite 590F, Spalte 2, Zeile 40 die Verbindung 2-Fluor-6-hydroxybenzonitril.

Derivate des 3-Fluorphenols sind wertvolle Zwischenprodukte zur Herstellung von Pflanzenschutzmitteln, Pharmazeutika und technischen Produkten wie z.B. Flüssigkristallen. Wegen der allgemeinen Bedeutung und der vielseitigen Verwendbarkeit dieser Stoffklasse stellt es eine lohnende Aufgabe dar, neue Verbindungen aus dieser Gruppe von Stoffen bereitzustellen, um das Spektrum ihrer Anwendungsmöglichkeiten nicht nur zu ergänzen, sondern auch durch eine Nuancierung stofflicher Eigenschaften zu bereichern und zu erweitern.

Diese Aufgabe wird gelöst durch Verbindungen der allgemeinen Formel (I) 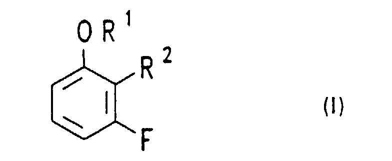 in welcher
- R¹: gleich CH₂-Phenyl ist, wobei die Phenylgruppe durch eine bis drei (C₁-C₄)-Alkoxygruppen, Fluor-, Chlor- oder Bromatome, Nitrogruppen, Cyanogruppen, Trifluormethylgruppen oder (C₁-C₄)-Alkoxycarbonylgruppen substituiert sein kann und
- R²: gleich -CN, -CONH₂, -NH₂, -NCO oder -NHCOOR³ ist, wobei R³ gleich (C₁-C₈)-Alkyl oder CH₂-Phenyl ist.

Als Alkylreste kommcn generell cyclische, geradkettige oder einfach oder mehrfach verzweigte Alkylreste, in Betracht. Beispiele hierfür sind die Methyl-, Ethyl-, n-Propyl-, i-Propyl-, n-Butyl-, i-Butyl-, n-Pentyl-, i-Pentyl-, n-Hexyl-, i-Hexyl-, n-Heptyl-, i-Heptyl-, n-Octyl-, i-Octyl-, 2-Chlorethyl-, 2-Bromethyl-, 2-Methoxyethyl-, 2-Ethoxyethyl-, 3-Methoxypropyl-, 3-Ethoxypropyl-, 4-Methoxybutyl-, 3-Chlorpropyl-, Cyclopropyl-, Cyclobutyl-, Cyclopentyl-, Cyclohexyl-, Methylcyclohexyl-.

Als Phenylreste kommen generell neben unsubstituiertem Phenyl ein- bis dreifach substituierte Phenylreste in Betracht.

Beispielsweise können hierbei genannt werden 2-Methylphenyl, 4-Methylphenyl, 2-Methoxyphenyl, 4-Methoxyphenyl, 2-Fluorphenyl, 3-Fluorphenyl, 4-Fluorphenyl, 4-Trifluormethylphenyl, 2,5-Dichlorphenyl, 2,4-Dichlorphenyl, 3-Nitrophenyl, 4-Cyanophenyl, 3,5-Dimethoxyphenyl, 2-Nitrophenyl, 2,4,5-Trichlorphenyl.

Wertvolle Zwischenprodukte sind auch die Verbindungen der Formel I worin R¹ für CH₂-Phenyl und R² für -CN, -CONH₂, NH₂ oder NHCOOR³ steht, insbesondere 2-Benzyloxy-6-fluorbenzamid, 2-Benzyloxy-6-fluoranilin, 2-Benzyloxy-6-fluor-N-benzyloxycarbonylanilin, 2-Benzyloxy-6-fluorbenzonitril.

Die vorliegende Erfindung betrifft ferner Verfahren zur Herstellung der erfindungsgemäßen Verbindungen.

So können Verbindungen der Formel I, in der R¹ gleich CH₂-Phenyl ist, wobei die Phenylgruppe durch eine bis drei (C₁-C₄)-Alkoxygruppen, Fluor-, Chlor- oder Bromatome, Nitrogruppen, Cyanogruppen, Trifluormethylgruppen oder (C₁-C₄)-Alkoxycarbonylgruppen substituiert sein und R² gleich -NH₂, -NHCOOR³ ist, hergestellt werden, indem man Verbindungen der allgemeinen Formel (II) 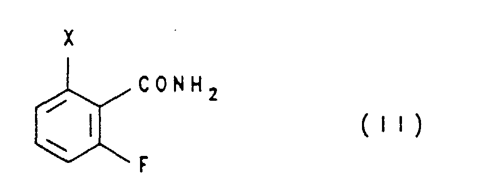 in der X OR¹ bedeutet, in wäßrig-alkalischem Medium mit Chlor, Brom, Natriumhypochlorit oder Natriumhypobromit in Gegenwart von Alkoholen bei Temperaturen von -15°C bis +80°C im Sinne eines Hofmann-Abbaus umsetzt.

In vielen Fällen hat es sich bewährt, als Alkoholkomponente Verbindungen der Formel HOR³ einzusetzen wobei R³ gleich (C₁-C₈)-Alkyl oder CH₂-Phenyl ist, wobei der Alkylrest oder die Phenylgruppe durch eine bis drei (C₁-C₄)-Alkoxygruppen, Fluor-, Chlor- oder Bromatome, Nitrogruppen, Cyanogruppen, Trifluormethylgruppen oder (C₁-C₄)-Alkoxycarbonylgruppen substituiert sein können.

Die bei der Umsetzung resultierende Verbindung der Formel (I') 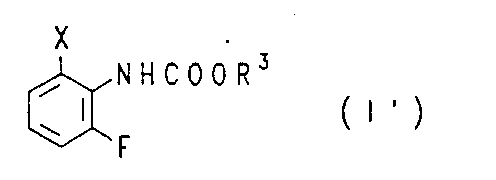 kann isoliert werden. Es ist jedoch auch möglich die Carbamatgruppe und/oder für X = OR¹ die Gruppe OR¹ hydrogenolytisch oder hydrolytisch zu spalten ohne eine Zwischenisolierung vorzunehmen. Ebenso können die Reaktionsbedingungen so gewählt werden, daß die Spaltung bereits weitgehend gleichzeitig zur Umlagerung stattfindet.

Die Verbindungen der Formel II werden hergestellt, indem man die entsprechenden Nitrile durch allgemein in der Literatur bekannte Methoden (J. March, Advanced Organic Chemistry [1985], 788) in die Benzamide überführt.

Bevorzugt ist hierbei die Umsetzung von 2,6-Difluorbenzonitril in wäßrig-alkalischem Medium mit Wasserstoffperoxid (JP-OS 60-132 942), gegebenenfalls entsprechend der vorliegenden Erfindung, unter Anwesenheit von etwa 1 bis etwa 20 Mol, bevorzugt etwa 0,1 bis 10 Mol, besonders bevorzugt zwischen etwa 1,05 Mol bis 5 Mol Alkohol pro 1 Mol 2,6-Difluorbenzonitril. Als Alkohole R¹-OH können hierbei Alkan-(C₁-C₈)-ole jeder Konstitution, die zusätzlich durch Alkoxy(C₁-C₄)gruppen, Fluor-, Chlor- oder Bromatome, Nitrogruppen, Cyanogruppen, Trifluormethylgruppen oder Alkoxy-(C1-C4)-carbonylgruppen substituiert sein können oder Phenylmethanole, wobei der Phenylrest durch Alkyl(C₁-C₄)gruppen, Alkoxy(C₁-C₄)gruppen, Fluor-, Chlor- oder Bromatome, Nitrogruppen, Cyanogruppen, Trifluormethylgruppen, Alkoxy-(C₁-C₄)-carbonylgruppen substituiert sein kann, verwendet werden, bevorzugt sind primäre Alkohole, besonders bevorzugt sind Methanol, Ethanol und Benzylalkohol.

2,6-Difluorbenzamid kann isoliert werden, es ist aber auch möglich im Eintopfverfahren sofort die Verbindungen der Formel II mit X = OR¹ zu erhalten. Dies kann vor allem durch Erhöhung der Reaktionstemperatur erreicht werden.

Man arbeitet bei Temperaturen zwischen etwa 0° und etwa 90°C, bevorzugt zwischen etwa 20° und etwa 70°C.

Setzt man zur Herstellung der Verbindungen der Formel II mit X = OR¹ zwischenisoliertes 2,6-Difluorbenzamid ein, arbeitet man erfindungsgemäß bevorzugt in überschüssigem Alkohol, wie oben beschrieben und setzt im ebenfalls bereits definierten Temperaturbereich mit etwa 1 Mol bis etwa 1,8 Mol, besonders bevorzugt mit etwa 1,1 Mol bis etwa 1,5 Mol alkalisch wirkenden Alkali- oder Erdalkalimetallverbindungen um. Solche alkalisch wirkenden Verbindungen sind zum Beispiel Hydroxide, Carbonate, Hydrogencarbonate, Phosphate, Hydrogenphosphate, Dihydrogenphosphate, Oxide oder ähnliche Verbindungen oder Mischungen derselben, bevorzugt sind Natrium- oder Kaliumhydroxid oder -carbonat. Die Reaktionszeiten betragen etwa 1 bis etwa 16 h, das Reaktionsprodukt wird im allgemeinen, falls Isolierung gewünscht ist, durch Verdünnen der Reaktionsmischung mit Wasser und Abfiltrieren isoliert (Extraktion, Kristallisation, Chromatographie).

Alternativ ist die Umsetzung mit den oben beschriebenen Mengen und Typen der alkalisch wirkenden Verbindungen und Alkoholen von 2,6-Difluorbenzonitril, wie weiter unten erläutert, oder 2,6-Difluorbenzamid in dipolar aprotischen Lösungsmitteln möglich. Als dipolar aprotische Lösungsmittel können Aceton, Tetrahydrofuran (THF), Acetonitril, 1,2-Dimethoxyethan (DMAc), N,N-Dimethylformamid (DMF), N-Methylpyrrolidon (NMP), Dimethylsulfoxid (DMSO), Dimethylsulfon, Diphenylsulfoxid, Diphenylsulfon, Tetramethylharnstoff, Tetra-n-butylharnstoff, 1,3-Dimethylimidazolidin-2-on (DMI) oder Mischungen derselben verwendet werden. Solche Lösungmittel werden in Mengen von etwa 50 bis 500 Massen-%, bezogen auf 2,6-Difluorbenzamid oder 2,6-Difluorbenzonitril, bevorzugt in Mengen zwischen etwa 80 bis etwa 250 Massen-% verwendet. Die Verwendung dieser Lösungsmittel erlaubt eine Abtrennung des bei der Reaktion gebildeten Alkali- oder Erdalkalimetallfluorides durch Filtration, das Produkt wird durch Zugabe von Wasser aus der Mutterlauge kristallisiert und das Lösungsmittel destillativ zurückgewonnen. Bei dieser Verfahrensvariante arbeitet man bei Reaktionstemperaturen zwischen etwa 30° und etwa 150°C, bevorzugt zwischen etwa 40° und etwa 100°C. Gegenüber wäßrigen Verfahrensvarianten besitzt diese Variante den Vorteil höherer Ausbeuten.

Bevorzugt ist die Herstellung von 2-Benzyloxy-6-fluorbenzamid.

Im Falle von 2-Benzyloxy-6-fluorbenzamid kann durch hydrogenolytische Abspaltung der Benzyloxygruppe 2-Hydroxy-6-fluorbenzamid erhalten werden.

In analoger Weise können Verbindungen der Formel I mit R² = CN erhalten werden. Hierzu wird 2,6-Difluorbenzonitril mit Alkoholen R³OH in wäßrig-alkalischem oder dipolar aprotischen Medium in Gegenwart von Alkalien umgesetzt.

Als Alkohole R¹-OH können hierbei Alkan-(C₁-C₈)-ole jeder Konstitution, die zusätzlich durch Alkoxy(C₁-C₄)gruppen, Fluor-, Chlor- oder Bromatome, Nitrogruppen, Cyanogruppen, Trifluormethylgruppen oder Alkoxy-(C₁-C₄)-carbonylgruppen substituiert sein können oder Phenylmethanole, wobei der Phenylrest durch Alkyl(C₁-C₄)gruppen, Alkoxy(C₁-C₄)gruppen, Fluor-, Chlor- oder Bromatome, Nitrogruppen, Cyanogruppen, Trifluormethylgruppen, Alkoxy-(C₁-C₄)-carbonylgruppen substituiert sein kann, verwendet werden, bevorzugt sind primäre Alkohole, besonders bevorzugt sind Methanol, Ethanol und Benzylalkohol.

Es ist auch möglich Verbindungen der Formel I mit R² = NH₂ im Eintopfverfahren zu erhalten, in dem man entweder 2,6-Difluorbenzonitril oder 2,6-Difluorbenzamid in wäßrig-alkalischem Medium mit Alkoholen zu Verbindungen der Formel II mit X = OR¹ umsetzt und diese dann nach Zwischenisolierung, vorzugsweise jedoch ohne Zwischenisolierung, im Eintopfverfahren mit Chlor, Brom, Natriumhypochlorit oder Natriumhypobromit bei Temperaturen von -15°C bis +80°C in Anwesenheit von Alkoholen im Sinne eines Hofmann-Abbaus umsetzt und anschließend gegebenenfalls gebildete Carbamate hydrolytisch oder hydrogenolytisch spaltet.

Auch hierbei können als Alkohole (C₁-C₈)-Alkanole oder Phenylmethanole, wobei der Alkylrest oder der Phenylrest durch eine bis drei (C₁-C₄)-Alkoxygruppen, Fluor-, Chlor- oder Bromatome, Nitrogruppen, Cyanogruppen, Trifluormethylgruppen oder (C₁-C₄)-Alkoxycarbonylgruppen substituiert sein kann, insbesondere primäre Alkohole eingesetzt werden.

In vielen Fällen hat sich die Verwendung von Methanol, Ethanol oder Benzylalkohol als günstig erwiesen.

Diese Alkohole werden in Mischung mit alkalisch wirkenden wäßrigen Lösungen wie oben beschrieben, bevorzugt Natrium- oder Kaliumhydroxid-Lösungen, eingesetzt. Man verwendet die wäßrig gelösten oder suspendierten alkalisch wirkenden Verbindungen in Mengen, bezogen auf abzubauendes Amid, zwischen etwa 1 Mol bis etwa 30 Mol, bevorzugt zwischen etwa 3 Mol bis etwa 15 Mol, besonders bevorzugt zwischen etwa 5 Mol und etwa 10 Mol. Die Konzentrationen der wäßrigen Lösungen hängen vom eingesetzten Amid ab, betragen jedoch typischerweise zwischen etwa 1 Mol/l bis etwa 20 Mol/l, bevorzugt zwischen etwa 3 Mol/l und etwa 10 Mol/l. Die Menge der alkalisch wirkenden wäßrigen Suspension wird in praxi so gewählt, daß die Rührbarkeit der Reaktionsmischung gewährleistet bleibt.

Man kann unter Verwendung von Hypohalogenit-Lösungen (Bleichlaugen) arbeiten, was der Dosierung von elementaren Halogen zu alkalisch wirkenden Lösungen vollkommen äquivalent ist. Zur Beschreibung der Reaktionsbedingungen ist die Angabe der eingesetzten Halogenmenge ausreichend, da sich in situ bei Kontakt von Halogen mit den eingesetzten alkalisch wirkenden wäßrigen Lösungen Hypohalogenitlösungen bilden. Man verwendet daher Chlor oder Brom in Mengen zwischen etwa 1 Mol bis etwa 5 Mol, bevorzugt etwa 1,01 Mol bis etwa 2 Mol, besonders bevorzugt zwischen etwa 1,02 Mol bis etwa 1,2 Mol, jeweils bezogen auf 1 Mol abzubauendes Amid. Verwendung von Chlor ist wegen der besseren technischen Verfügbarkeit bevorzugt. Das Halogen kann zugetropft (Brom) oder gasförmig eingeleitet (Chlor) werden. Je nach Reaktionstemperatur kommen dabei im Labormaßstab Dosierzeiten zwischen etwa 0,5 h bis etwa 8 h, bevorzugt etwa 1 h bis etwa 4 h in Frage. Im technischen Maßstab müssen die Dosierzeiten aufgrund der Exothermie der Umsetzung den vorhandenen Kühlkapazitäten angepaßt werden.

Man arbeitet bei der Umsetzung im Sinne des Hofmann-Abbaus bei Temperaturen zwischen etwa -15°C und etwa 100°C, bevorzugt zwischen etwa 0°C und etwa 60°C, besonders bevorzugt zwischen etwa 10°C und etwa 40°C.

Dosiert man Bleichlaugen, also wäßrige Hypohalogenit-Lösungen, was im allgemeinen gegenüber elementarem Halogen Handhabungsvorteile aufweist, so verwendet man Lösungen mit einem Gehalt an aktivem Chlor von etwa 30 bis etwa 250 g pro kg Lösung, bevorzugt zwischen etwa 100 und etwa 160 g aktiven Chlor pro kg Lösung bzw. etwa 60 bis etwa 550 g aktivem Brom pro kg Lösung, bevorzugt zwischen etwa 200 bis etwa 350 g pro kg Lösung. Diese Lösungen können durch Dosieren der entsprechenden Mengen Chlor oder Brom in wäßrige, alkalisch wirkende Lösungen erhalten werden.

Das primäre Reaktionsprodukt aus der Umsetzung im Sinne eines Hofmann-Abbaus ist bei der erfindungsgemäßen Variante vermutlich das Alkyl-carbamat des gewünschten Anilins, da das als Zwischenstufe auftretende Isocyanat wahrscheinlich durch die unter Reaktionsbedingungen nucleophilen Alkoholate abgefangen wird.

Die Aniline können nach im Prinzip literaturbekannten Verfahren aus den Carbamaten erhalten werden (T.W. Greene, P.G.M. Wuts, Protective Groups in Organic Chemistry (1991), 317-348). In der Regel gelingt die Freisetzung der Aminogruppe durch Erhitzen der erfindungsgemäßen Reaktionsmischung, nachdem eventuelle Chlor- oder Bromüberschüsse vernichtet worden sind. Gegebenenfalls substituierte Benzylcarbamate sind unter alkalischen Bedingungen in weniger als 10 h bei 80 bis 100°C zu hydrolysieren, während einfache Alkylcarbamate bei denselben Temperaturen Reaktionszeiten bis zu 96 h benötigen. Besonders bevorzugt ist daher die Verwendung von gegebenenfalls substituiertem Benzylalkohol, da sich die entstehenden Benzylcarbamate ohne großen Aufwand und daher kostengünstig durch Hydrierung spalten lassen (T.W. Greene et al., loc. cit., 335-341). Die hydrogenolytische Spaltung wird vorteilhaft in Gegenwart von Übergangsmetallkatalysatoren, insbesondere Platin-, Nickel- oder Palladium-Katalysatoren durchgeführt. Die Reaktionsprodukte können im allgemeinen einfach durch Phasentrennung isoliert werden, wobei Mischungen mit den eingesetzten Alkoholen anfallen. Die Phasentrennung kann durch Zusatz von zusätzlichen Lösungsmitteln wie zum Beispiel Toluol oder Xylol verbessert oder herbeigeführt werden.

Die anfallenden Lösungen der freien Amine können, sofern sie Verbindungen enthalten, die Benzylgruppen aufweisen, durch im allgemeinen literaturbekannte Methoden hydriert werden (T.W. Greene et al., loc. cit., 47-68, 156-160, 335-341). Bevorzugt ist die Hydrierung unter Verwendung von Wasserstoff-Gas in Anwesenheit von Übergangsmetall-Katalysatoren, bevorzugt Palladium-Katalysatoren, besonders bevorzugt von Palladium auf Aktivkohle. Alternativ möglich ist die Verwendung der Methode der sogenannten Transfer-Hydrierung zur Synthese der neuen Zwischenprodukte (T.W. Greene et al., loc. cit., 156-160). Neben Palladium kommen als eingesetzte Übergangsmetalle insbesondere Nickel oder Platin in Frage. Die Hydrierungen verlaufen glatt unter Wasserstoffdruck zwischen etwa 1,1 bar und etwa 100 bar bei Temperaturen zwischen etwa 10° und etwa 80°C in niederen aliphatischen Alkoholen oder einfachen aromatischen oder aliphatischen Kohlenwasserstoffen wie zum Beispiel Hexan, Methylcyclohexan, Toluol, Xylol, Methanol, Butanol, Isopropanol oder Ethanol als Lösungsmittel oder Mischungen derer. Die Edelmetallkatalysatoren werden in Mengen zwischen etwa 0,05 bis etwa 3, bevorzugt etwa 0,3 bis etwa 1 Massen-% (als reines Übergangsmetall gerechnet) eingesetzt. Die Konzentration des Endproduktes (insbesondere 2-Amino-3-fluor-phenol) in der Mutterlauge der Hydrierung beträgt typischerweise zwischen etwa 10 bis etwa 500, bevorzugt zwischen etwa 100 bis etwa 300 g/l. Setzt man dabei die unsubstituierten Benzylverbindungen ein, so erhält man bei der Hydrierung die freien Hydroxy-gruppen und Toluol. Daher ist Toluol als Lösungsmittel für die Hydrierung bevorzugt. Das Endprodukt, 2-Amino-3-fluorphenol, kann durch Einengen der vom Katalysator gegebenenfalls heiß filtrierten Lösungen und anschließende Filtration gewonnen werden. Dabei bietet es sich an, unter Anwesenheit von oxidationsverhindernden Zusätzen wie Hydrazin oder Hydraziniumsalzen oder 2,6-Di-tert.-butyl-4-methylphenol zu arbeiten, da das Endprodukt hohe Labilität gegen Luftsauerstoff, besonders beim Erwärmen zeigt.

Besonders bevorzugt ist die Verwendung von Benzylalkohol in den Reaktionsschritten, die unter Anwesenheit von Alkoholen verlaufen, im erfindungsgemäßen Verfahren.

Die Verbindungen der Formel I, insbesondere Verbindungen, worin R¹ = CH₂-Phenyl, R² = -CN, -CONH₂, -NHCOOR³, NH₂ mit R³ Methyl, Ethyl, CH₂-Phenyl bedeuten, können zur Herstellung von flüssigkristallinen Verbindungen, Pflanzenschutzmitteln und Pharmazeutika verwendet werden.

Die folgenden Beispiele erläutern die Erfindung, ohne sie zu beschränken.

### Beispiel 1

### Herstellung von 2-Benzyloxy-6-fluoranilin

In 450 g Wasser werden 180 g Benzylalkohol und 269 g (6,725 Mol) Natriumhydroxid eingetragen. Bei 20°C gibt man 220 g (0,9 Mol) 2-Benzyloxy-6-fluorbenzamid und leitet bei 40°C unter guter Rührung Chlor ein (15 l/h). Die Umsetzung wird gaschromatographisch überwacht und bei vollständigem Umsatz nach 2,5 h abgebrochen. Man heizt die wäßrige Mutterlauge 3 h auf 95°C, nachdem ein eventueller Überschuß Chlor zerstört worden ist (Natriumsulfit).

Durch Extraktion mit MTBE, Trocknen über Magnesiumsulfat und Entfernen des Lösungsmittels kann an dieser Stelle 2-Benzyloxy-6-fluoranilin als leicht braun gefärbtes zähes Öl gewonnen werden (siehe auch Beispiel 3), auf diesen Schritt wird jedoch hier verzichtet. Das Reaktionsprodukt kann, wie nachfolgend unter Verfahrensweise a und b beschrieben, zu 2-Amino-3-fluorphenol umgesetzt werden.

### Verfahrensweise a

Die organische Phase wird von der wäßrigen Phase getrennt, in 200 ml Methanol aufgenommen und danach mit 5 g Pd/C (5 % Pd, 50 %ig feucht) solange unter H₂-Atmosphäre (schwacher Überdruck) kräftig gerührt (15 h), bis keine Verbindungen mit Benzyloxygruppierungen mehr nachzuweisen sind. Man filtriert den Katalysator ab und wäscht mit Methanol nach. Unter Inertgas wird das Methanol im wesentlichen abdestilliert und 300 g Toluol zugegeben. Nach dem Kühlen (0°C) kann man 60,9 g (0,48 Mol, 53 %) nach dem Trocknen hellbraun bis dunkelgrau gefärbtes 2-Amino-3-fluorphenol erhalten (Gehalt (GC): 100 %). In der schwarzen Mutterlauge sind weitere 24,2 g (0,19 Mol, 21 %) Produkt enthalten, wie durch quantitative Gaschromatographie ermittelt wird. Die Mutterlauge wird bei weiteren Ansätzen wieder verwendet.

### Verfahrensweise b

Nach einigen Minuten ohne Rührung können die Phasen nach Zugabe von 200 g Toluol getrennt werden, die organische Phase wird mit 300 g Methanol aufgefüllt und mittels 5 g Pd/C (5 % Pd, 50 %ig feucht) unter H₂-Atmosphäre (schwacher Überdruck) kräftig gerührt (20 h). Die GC-Analyse weist Lösungsmittel und neben untergeordneten Mengen von Nebenprodukten 2-Amino-3-fluorphenol als Hauptkomponente aus. Man filtriert den Katalysator ab und wäscht mit Methanol nach. Unter Inertgas wird das Methanol aus dem Filtrat im wesentlichen abdestilliert und 300 g Toluol zugegeben. Nach dem Kühlen (0°C) und nach dem Trocknen kann man 65,3 g (0,51 Mol, 57 %) hellbraun gefärbtes 2-Amino-3-fluorphenol erhalten (Gehalt (GC): 100 %). In der Mutterlauge sind weitere 28,2 g (0,22 Mol, 25 %) Produkt enthalten, wie durch quantitative Gaschromatographie ermittelt wurde. Die Mutterlauge wird bei weiteren Ansätzen wieder verwendet.
2-Amino-3-fluorphenol:
   Schmp. 124-126°C; 126,3°C (DSC)
   Sdp. 250,7°C (DSC)
   Wasserlöslichkeit < 15 g/l
   Schüttgewicht ca. 0,2 g/cm³
¹H-NMR [DMSO-d₆/TMS, ppm]:
   δ = 6,39 (C) (ddd, 1H, J_{BC}= 8,28 Hz, J_{CD} 6,36 Hz, J_{AC}= 7,83 Hz, Ar-H⁵) 6,50 (B) (ddd, 1H, J_{BC}= 8,28 Hz, J_{BD}= 8,30 Hz, J_{AB} 1,48 Hz, Ar-H⁴) 6,52 (A) (ddd, 1H, J_{AC}= 7,83 Hz, J_{AD} 2,30 Hz, J_{AB} 1,48 Hz, Ar-H⁶)
¹⁹F-NMR [DMSO-d₆/CFCl₃, ppm]:
   δ = -133,94 (D) (ddd, 1F, J_{BD}= 6,36 Hz, J_{AD}= 2,30 Hz, J_{CD} 6,36 Hz, Ar-F³)
¹³C-NMR [DMSO-d₆/TMS, ppm]:
   δ = 106,1 (J= 19,1 Hz, Ar-C²), 110,5 (Ar-C⁶), 115,4 (J= 9,4 Hz, Ar-C¹), 124,5 (J= 14,8 Hz, Ar-C⁴), 146,1 (J= 7,9 Hz, Ar-C⁵), 151,5 (J =-234,2 Hz, Ar-C³)
MS: m/z (%) = 51 (14), 52 (16), 57 (2), 63 (3), 70 (6), 71 (7), 78 (7), 79 (13), 80 (5), 81 (4), 82 (8), 98 (57), 99 (5), 126 (11), 127 (100, M⁺), 128 (7)

### Beispiel 2

### A. Herstellung von 2-Benzyloxy-6-fluorbenzamid in wäßrigem Medium

(1) In 232,3 g (2,15 Mol) Benzylalkohol werden 261,2 g (1,66 Mol) 2,6-Difluorbenzamid suspendiert, die Suspension auf 55°C geheizt und in 2 h 233,3 g (2,08 Mol) 50 %ige Kaliumhydroxidlösung zugetropft. Nach dieser Zeit wird die Temperatur auf 60°C erhöht und 3 h weitergerührt.
(2) Die gesamte Reaktionsmischung wird unter Rühren in 1900 g Wasser einlaufen gelassen und der ausgefallene Feststoff abgesaugt. Es wird 3mal mit je 200 ml Wasser gewaschen und das Produkt im Vakuum bei 70°C getrocknet. Aus 381 g Feuchtprodukt werden 297,7 g (1,21 Mol, 73 %) 2-Benzyloxy-6-fluorbenzamid als leicht gelbliches Pulver erhalten (Reingehalt (GC) > 98 %).

### B. Herstellung in dipolar aprotischen Lösungsmitteln

In 230 g N,N-Dimethylacetamid (DMAc) werden 165,6 g (1,2 Mol) Kaliumcarbonat suspendiert und 113,4 g (1,05 Mol) Benzylalkohol zugegeben. Man erhitzt nach Zugabe von 157,1 g (1 Mol) 2,6-Difluorbenzamid 24 h auf 130°C und filtriert vom ausgefallenen feinen Salz ab, das mit 250 g DMAc in mehreren Portionen gewaschen wird (184,5 g trocken). Die organische Phase (658,6 g) wird in 1100 g Wasser unter Rühren eingetropft, wobei das Reaktionsprodukt auskristallisiert. Man filtriert und wäscht den Filterkuchen dreimal mit je 150 g Wasser. Man erhält 405 g Feuchtprodukt und nach dem Trocknen 170,7 g (0,696 Mol, 70 %) 2-Benzyloxy-6-fluorbenzamid als gelbbeigefarbenes Pulver.
2-Benzyloxy-6-fluorbenzamid:
   Schmp. 142,6°C (DSC)
¹H-NMR [DMSO-d₆/TMS, ppm]:
   δ = 5,15 (1) (s, 2H, Ar-CH₂)
   6,81 (G) (ddd, 1H, J_{EG}= 8,2 Hz, J_{FG}= 0,5 Hz, J_{GK}= 8,9 Hz, Ar-H⁵) 6,95 (F) (d(dd), 1H, J_{EF}= 8,5 Hz, J_{FG}= 0,5 Hz, J_{FK}= 0,5 Hz, Ar-H³) 7,31 (H) (tm, 2H, Ar(Benzyl)-H^{3,5})
   7,33 (E) (ddd, 1H, J_{EF}= 8,5 Hz, J_{EG}= 8,2 Hz, J_{EK}= -6,9 Hz, Ar-H⁴) 7,38 (D) (tm, 1H, Ar(Benzyl)-H⁴)
   7,45 (C) (dm, 2H, Ar(Benzyl)-H^{2,6})
   7,49 (B) (s(br), 1H, Ar-NH₂^{cis/trans})
   7,80 (A) (s(br), 1H, Ar-NH₂^{cis/trans})
¹⁹F-NMR [DMSO-d₆/CFCl₃, ppm]:
   δ = -116,40 (K) (ddd, 1F, J_{EK}= -6,9 Hz, J_{FK}= 0,5 Hz, J_{GK}= 8,9 Hz, Ar-F⁶)
MS: m/z (%) = 63 (5), 65 (17), 91 (100), 92 (8), 98 (3), 110 (6), 123 (4), 138 (3), 139 (5), 155 (1), 199 (1), 200 (1), 228 (20), 229 (3), 245 (8,1, M⁺) 246 (1)

### Beispiel 3

### 2-Benzyloxy-6-fluoranilin

Es werden 120 g Methanol, 70 g Wasser, 30 g (0,75 Mol) Natriumhydroxid und 24,5 g (0,1 Mol) 2-Benzyloxy-6-fluorbenzamid vorgelegt und auf 40°C erhitzt. Man leitet Chlor ein (4 l/h), wobei sich die farblose Suspension nach kurzer Zeit leicht bräunlich verfärbt und die Heizung entfernt werden kann, da sich die Temperatur durch die Exothermie der Umsetzung hält. Nach 25 min ist die Umsetzung beendet, wie durch GC nachgewiesen werden kann. Statt der anfänglich eingesetzten Suspension wird eine klare Lösung erhalten. Man destilliert das Methanol im schwachen Vakuum ab (50°C) und erhitzt die erhaltene Suspension von 2-Benzyloxy-6-fluor-N-carbomethoxy-anilin 48 h auf 100°C. Nach dem Abkühlen werden 50 g Toluol zugegeben, die Phasen getrennt und die organische Phase mit 2 g MgSO₄ und 1 g Aktivkohle versetzt und einige Stunden gerührt. Nach Filtration und Entfernen des Lösungsmittels am Rotationsverdampfer erhält man 19,8 g (91 mMol, 91 %) 2-Benzyloxy-6-fluoranilin als bräunliches, klares Öl, das für weitere Umsetzungen eine ausgezeichnete Reinheit (GC: > 95 %) aufweist.
2-Benzyloxy-6-fluoranilin:
   ¹H-NMR [DMSO-d₆/TMS, ppm]:
   δ = 4,57 (H) (s(br), 2H, Ar-NH₂) 5,13 (G) (s, 2H, Ar(Benzyl)-CH₂) 6,51 (F) (ddd, 1H, J_{EF}= 8,35 Hz, J_{DF}= 8,15 Hz, J_{IF}= 6,35 Hz, Ar-H⁴) 6,68 (E) (ddd, 1H, J_{EF}= 8,35 Hz, J_{DE}= 1,30 Hz, J_{EI}= 10,6 Hz, Ar-H⁵) 6,77 (D) (ddd, 1H, J_{ED}= 1,30 Hz, J_{DF}= 8,15 Hz, J_{DI}= 1,20 Hz, Ar-H³) 7,21 (C) (tm, 1H, Ar(Benzyl)-H⁴) 7,39 (C) (tm, 2H, Ar(Benzyl)-H^{3,5}) 7,48 (B) (dm, 2H, Ar(Benzyl)-H^{2,6})
¹⁹F-NMR [DMSO-d₆/CFCl₃, ppm]:
   δ = -133,77 (1) (ddd, 1F, J_{EI}= 10,6 Hz, J_{FI}= 6,35 Hz, J_{DI}= 1,20 Hz, Ar-F⁶)
MS: m/z (%) = 51 (9), 63 (4), 65 (16), 91 (100), 92 (8), 98 (16), 126 (10), 138 (0,5), 217 (17,6, M⁺), 218 (2,6)

2-Benzyloxy-6-fluor-N-carbomethoxy-anilin kann isoliert werden, wenn man wie oben angegeben verfährt, jedoch auf die 48-stündige Hydrolyse des Zwischenproduktes verzichtet und dieses nach üblichen Methoden (insbesondere Filtration und Umkristallisation) isoliert und reinigt.
MS: m/z (%) = 51 (3,2), 59 (1,7), 63 (3,0) 65 (14,0) 70 (3,2), 83 (1,6), 89 (2,5), 91 (100), 92 (9,7), 112 (1,2) 152 (1,6), 153 (1,3), 216 (1,6) 243 (8,0), 244 (1,3), 275 (15,0, M⁺), 276 (2,6)

2-Benzyloxy-6-fluor-phenylisocyanat entsteht als Zwischenprodukt bei der Synthese und kann, sofern gewünscht, isoliert werden.
MS: m/z (%) = 51 (4,2), 63 (3,1), 65 (15,1), 76 (5,3), 89 (3,2), 91 (100), 92 (8,2), 96 (2,6), 108 (1,1), 152 (8,9), 124 (3,7), 152 (1,6), 243 (15,9, M⁺)

### Beispiel 4

### 2-Benzyloxy-6-fluorbenzonitril

Es werden 100 g Benzylalkohol und 55,6 g (0,4 Mol) 2,6-Difluorbenzonitril vorgelegt und auf 40°C erwärmt. Dann wird unter Rühren 49,2 g (0,44 Mol) 50 %ige Kaliumhydroxid-Lösung so zugetropft, daß die Temperatur gehalten werden kann (Kühlung). Nach 4 h verdünnt man die Mischung mit 200 ml Wasser, saugt ausgefallenes Produkt ab und versetzt die Mutterlauge im Scheidetrichter mit 50 g Toluol. Die Auswaage an Feuchtprodukt beträgt 62,0 g, nach dem Trocknen verbleiben 56,2 g (0,247 Mol, 62 %) 2-Benzyloxy-6-fluorbenzonitril (Reingehalt (GC) 99,3 %) in Form eines leicht gelblichen Feststoffes. Aus der organischen Phase wird durch Entfernen des Toluols im Vakuum (bis 190°C) 31,5 g 92,4 %iges Material (29,1 g, 0,128 Mol, 32 %) erhalten. Die Ausbeute an reinem 2-Benzyloxy-6-fluorbenzonitril beträgt nach Umlösung der Rohprodukte aus Ethanol/Wasser 81,2 g (0,36 Mol, 90 %), das Produkt fällt in farblosen Flittern an.
2-Benzyloxy-6-fluorbenzonitril
   Schmp. 71,9°C (DSC)
MS: m/z (%) = 50 (3), 51 (6), 57 (3), 63 (7), 65 (27), 89 (5), 91 (100), 92 (15), 108 (7), 120 (1), 136 (1), 170 (1), 227 (M⁺, 13,7), 228 (2,4)

## Patentansprüche

1. Verbindungen der allgemeinen Formel (I) 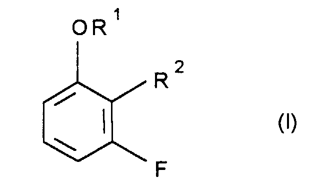 in welcher R¹ gleich CH₂-Phenyl ist, wobei die Phenylgruppe durch eine bis drei (C₁-C₄)-Alkoxygruppen, Fluor-, Chlor- oder Bromatome, Nitrogruppen, Cyanogruppen, Trifluormethylgruppen oder (C₁-C₄)-Alkoxycarbonylgruppen substituiert sein kann und R² gleich -CN, -CONH₂, -NH₂, -NCO oder -NHCOOR³ ist, wobei R³ gleich (C₁-C₈)-Alkyl oder CH₂-Phenyl ist.

2. Verbindungen nach Anspruch 1, worin R¹ gleich CH₂-Phenyl ist und R² -CN, -CONH₂, -NH₂ oder -NHCOOR³ ist.

3. Verbindungen der allgemeinen Formel (I) worin R³ CH₂-Phenyl ist.

4. Verbindungen der allgemeinen Formel (I) gemäß Anspruch 1 aus der Gruppe 2-Benzyloxy-6-fluorbenzamid, 2-Benzyloxy-6-fluoranilin, 2-Benzyloxy-6-fluor-N-benzyloxycarbonylanilin, 2-Benzyloxy-6-fluor-benzonitril.

5. Verfahren zur Herstellung von Verbindungen der Formel (I) gemäß Anspruch 1, in der R¹ die Bedeutung gemäß Anspruch 1 hat und R² für -NH₂, -NHCOOR³ steht, dadurch gekennzeichnet, daß man Verbindungen der allgemeinen Formel (II) 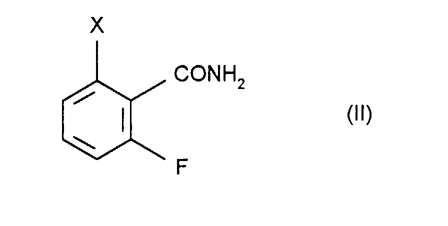 in der X gleich OR¹ bedeutet, in wäßrig-alkalischem Medium mit Chlor, Brom, Natriumhypochlorit oder Natriumhypobromit in Gegenwart von Alkoholen bei Temperaturen von -15°C bis +80°C im Sinne eines Hofmann-Abbaus umsetzt.

6. Verfahren nach Anspruch 5, dadurch gekennzeichnet, daß die Umsetzung in Gegenwart eines Alkohols HOR³ erfolgt, wobei R³ die gleiche Bedeutung wie in Anspruch 1 hat.

7. Verfahren nach Anspruch 6, dadurch gekennzeichnet, daß die dabei resultierende Verbindung der allgemeinen Formel (I') 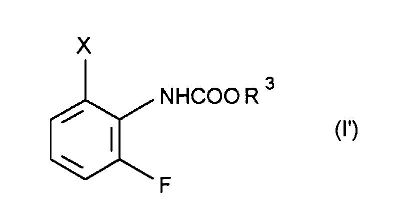 entweder isoliert oder die Carbamatgruppe und/oder für X = OR¹ die Gruppe OR¹ hydrogenolytisch oder hydrolytisch gespalten wird.

8. Verfahren nach mindestens einem der Ansprüche 5 bis 7, dadurch gekennzeichnet, daß man zur Herstellung der Verbindungen (II) von 2,6-Difluorbenzonitril ausgeht und die Nitrilgruppe in an sich bekannter Weise in die Amidgruppe überführt und bei Verbindungen in denen X gleich OR¹ ist, das entsprechende F-Atom vor, während oder nach dieser Überführung gegen die Gruppe OR¹ austauscht.

9. Verfahren nach Anspruch 8, dadurch gekennzeichnet, daß der Austausch des Fluoratoms gegen eine Benzyloxygruppe gleichzeitig mit der Überführung der Nitril- in die Amidgruppe in der Weise erfolgt, daß das 2,6-Difluorbenzonitril in Gegenwart eines Alkohols HOR³, wobei R³ die gleiche Bedeutung wie in Anspruch 1 hat und vorzugsweise einen Benzylrest darstellt, in das entsprechende Benzamid überführt wird und die OR¹-Gruppe (R¹ hier von Wasserstoff verschieden) gegebenenfalls anschließend hydrogenolytisch gespalten wird.

10. Verfahren nach Anspruch 8, dadurch gekennzeichnet, daß man das 2,6-Difluorbenzonitril zur Herstellung der Verbindung der Formel (I) mit R² = CN mit einem Alkohol HOR³, wobei R³ die gleiche Bedeutung wie in Anspruch 1 besitzt, in wäßrig-alkalischem Medium oder in einem dipolar aprotischen Lösungsmittel in Gegenwart von Alkalien umsetzt.

11. Verfahren zur Herstellung der Verbindungen der Formel (I) gemäß Anspruch 1, in der R² gleich NH₂ ist, dadurch gekennzeichnet, daß man entweder 2,6-Difluorbenzonitril oder 2,6-Difluorbenzamid in wäßrig-alkalischem Medium mit Alkoholen zu Verbindungen der Formel II mit X = OR¹ umsetzt und diese dann nach Zwischenisolierung, vorzugsweise jedoch ohne Zwischenisolierung im Eintopfverfahren, mit Chlor, Brom, Natriumhypochlorit oder Natriumhypobromit bei Temperaturen von -15°C bis +80°C in Anwesenheit von Alkoholen im Sinne eines Hofmann-Abbaus umsetzt und anschließend gegebenenfalls gebildete Carbamate hydrolytisch oder hydrogenolytisch spaltet.

12. Verfahren nach Anspruch 11, dadurch gekennzeichnet, daß man als Alkohole Phenylmethanole, wobei der Alkylrest oder der Phenylrest durch eine bis drei (C₁-C₄)-Alkoxygruppen, Fluor, Chlor oder Bromatome, Nitrogruppen, Cyanogruppen, Trifluormethylgruppen oder (C₁-C₄)-Alkoxycarbonylgruppen substituiert sein kann, einsetzt.

13. Verfahren nach Anspruch 11 oder 12, dadurch gekennzeichnet, daß es sich bei dem Alkohol um Benzylalkohol handelt.

14. Verfahren nach mindestens einem der Ansprüche 11 bis 13, dadurch gekennzeichnet, daß man die Umsetzungen zu den 2-Benzyloxy-6-fluorbenzamiden in wäßrig-alkalischem Medium oder in einem oder mehreren dipolar aprotischen Lösungsmitteln in Gegenwart von Alkalien durchführt.

15. Verfahren nach mindestens einem der Ansprüche 11 bis 13, dadurch gekennzeichnet, daß die Umsetzung zu den Verbindungen der Formel (II) mit X OR¹ bei Temperaturen von 0°C bis 90°C, vorzugsweise 20°C bis 70°C erfolgt.

16. Verfahren nach Anspruch 14, dadurch gekennzeichnet, daß die Umsetzung bei Temperaturen von 30°C bis 150°C, vorzugsweise 40°C bis 100°C erfolgt.

17. Verfahren nach mindestens einem der Ansprüche 11 bis 16, dadurch gekennzeichnet, daß man bei den nach dem Hofmann-Abbau erhaltenen Verbindungen die darin enthaltenen Carbamatgruppen, sowie gegebenenfalls auch die OR¹-Gruppen, hydrogenolytisch und/oder hydrolytisch spaltet.

18. Verfahren nach Anspruch 17, dadurch gekennzeichnet, daß man die hydrogenolytische Spaltung in Gegenwart von Übergangsmetallkatalysatoren insbesondere Platin-, Nickel- oder Palladiumkatalysatoren, durchführt.

19. Verwendung der Verbindungen der Formel (I), gemäß Anspruch 1 insbesondere Verbindungen, worin
R¹ = CH₂-Phenyl und
R² = -CN, -CONH₂, -NH₂, -NHCOOR³ mit R³ = Methyl, Ethyl, CH₂-Phenyl bedeuten, zur Herstellung von flüssigkristallinen Verbindungen, Pflanzenschutzmitteln oder Pharmazeutica.

## Claims

1. A compound of the formula (I) 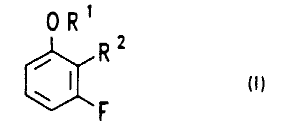 in which R¹ is CH₂-phenyl, where the phenyl group can be substituted by one to three (C₁-C₄)-alkoxy groups, fluorine, chlorine or bromine atoms, nitro groups, cyano groups, trifluoromethyl groups or (C₁-C₄)-alkoxycarbonyl groups, and R² is -CN, -CONH₂, -NH₂, -NCO or -NHCOOR³, where R³ is (C₁-C₈)-alkyl or CH₂-phenyl.

2. A compound as claimed in claim 1, wherein R¹ is CH₂-phenyl and R² is -CN, -CONH₂, -NH₂ or -NHCOOR³.

3. A compound of the formula (I), wherein R³ is CH₂-phenyl.

4. A compound of the formula (I) as claimed in claim 1 from the group comprising 2-benzyloxy-6-fluorobenzamide, 2-benzyloxy-6-fluoroaniline, 2-benzyloxy-6-fluoro-N-benzyloxycarbonylaniline and 2-benzyloxy-6-fluorobenzonitrile.

5. A process for preparing compounds of the formula (I) as claimed in claim 1, in which R¹ has the meaning as claimed in claim 1 and R² is -NH₂ or -NHCOOR³, wherein compounds of the formula (II) 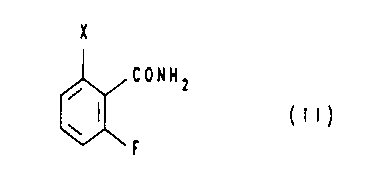 in which X is OR¹, are reacted, in aqueous-alkaline medium, with chlorine, bromine, sodium hypochlorite or sodium hypobromite in the presence of alcohols and at temperatures of from -15°C to +80°C, in the sense of a Hofmann degradation.

6. The process as claimed in claim 5, wherein the reaction is effected in the presence of an alcohol, HOR³, where R³ has the same meaning as in claim 1.

7. The process as claimed in claim 6, wherein the compound of the formula (I') 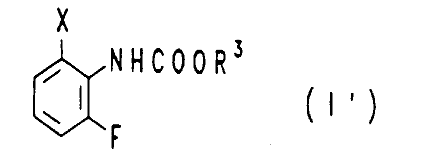 resulting therefrom is either isolated, or the carbamate group and/or, for X = OR¹, the group OR¹ is cleaved hydrogenolytically or hydrolytically.

8. The process as claimed in at least one of claims 5 to 7, wherein 2,6-difluorobenzonitrile is used as the starting compound for preparing the compounds (II) and the nitrile group is converted, in a manner known per se, into the amide group, and, in the case of compounds in which X is OR¹, the corresponding F atom is replaced by the OR¹ group before, during or after this conversion.

9. The process as claimed in claim 8, wherein the replacement of the fluorine atom by a benzyloxy group is effected at the same time as the conversion of the nitrile group into the amide group in such a way that the 2,6-difluorobenzonitrile is converted, in the presence of an alcohol, HOR³, where R³ has the same meaning as in claim 1 and preferably represents a benzyl radical, into the corresponding benzamide, and the OR¹ group (here R¹ is different from hydrogen) is subsequently, where appropriate, cleaved hydrogenolytically.

10. The process as claimed in claim 8, wherein the 2,6-difluorobenzonitrile for preparing the compound of the formula (I) having R² = CN is reacted with an alcohol, HOR³, where R³ possesses the same meaning as in claim 1, in aqueous-alkaline medium or in a dipolar, aprotic solvent in the presence of alkalis.

11. A process for preparing the compounds of the formula (I) as claimed in claim 1, in which R² is NH2, wherein either 2,6-difluorobenzonitrile or 2,6-difluorobenzamide is reacted, in aqueous-alkaline medium, with alcohols to form compounds of the formula II having X = OR¹, and these latter are then, after intermediate isolation, but preferably without any intermediate isolation, reacted, in a one-pot process, with chlorine, bromine, sodium hypochlorite or sodium hypobromite, at temperatures of from -15°C to +80°C and in the presence of alcohols, in the sense of a Hofmann degradation, and, where appropriate, carbamates which have been formed are subsequently cleaved hydrolytically or hydrogenolytically.

12. The process as claimed in claim 11, wherein phenylmethanols, where the alkyl radical or the phenyl radical can be substituted by one to three (C₁-C₄)-alkoxy groups, fluorine, chlorine or bromine atoms, nitro groups, cyano groups, trifluoromethyl groups or (C₁-C₄) -alkoxycarbonyl groups, are employed as alcohols.

13. The process as claimed in claim 11 or 12, wherein the alcohol is benzyl alcohol.

14. The process as claimed in at least one of claims 11 to 13, wherein the reactions to form the 2-benzyloxy-6-fluorobenzamides are carried out in aqueous-alkaline medium or in one or more dipolar, aprotic solvents in the presence of alkalis.

15. The process as claimed in at least one of claims 11 to 13, wherein the reaction to form the compounds of the formula (II) having X = OR¹ is effected at temperatures of from 0°C to 90°C, preferably of from 20°C to 70°C.

16. The process as claimed in claim 14, wherein the reaction is effected at temperatures of from 30°C to 150°C, preferably of from 40°C to 100°C.

17. The process as claimed in at least one of claims 11 to 16, wherein the carbamate groups, and, where appropriate, the OR¹ groups as well, contained in the compounds obtained by the Hofmann degradation, are cleaved hydrogenolytically and/or hydrolytically.

18. The process as claimed in claim 17, wherein the hydrogenolytic cleavage is carried out in the presence of transition metal catalysts, in particular platinum, nickel or palladium catalysts.

19. Use of the compounds of the formula (I), as claimed in claim 1, in particular compounds in which
R¹ is CH₂-phenyl, and
R² is -CN, -CONH₂, -NH₂ or -NHCOOR³, with R³ being methyl, ethyl or CH₂-phenyl,
for preparing liquid-crystalline compounds, plant protection agents or pharmaceuticals.

## Revendications

1. Composés de formule générale (I) : 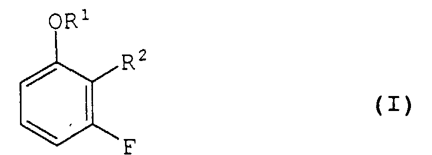 dans laquelle R¹ est CH₂-phényle, le groupe phényle pouvant être substitué par un à trois groupes alcoxy en C₁ à C₄, atomes de fluor, chlore ou brome, groupes nitro, groupes cyano, groupes trifluorométhyle ou groupes alcoxy (en C₁ à C₄)- carbonyle ; et R² est -CN, -CONH₂, -NH₂, -NCO ou -NHCOOR³, dans lequel R³ est un alkyle en C₁ à C₈ ou CH₂-phényle.

2. Composés selon la revendication 1, dans lesquels R¹ est CH₂-phényle et R² est -CN, -CONH₂, -NH₂ ou -NHCOOR³.

3. Composés de formule générale (I), dans lesquels R³ est CH₂-phényle.

4. Composés de formule générale (I) selon la revendication 1, choisis dans le groupe des composés suivants : 2-benzyloxy-6-fluorobenzamide, 2-benzyloxy-6-fluoraniline, 2-benzyloxy-6-fluoro-N-benzyloxycarbonylaniline, 2-benzyloxy-6-fluorobenzonitrile.

5. Procédé de préparation de composés de formule (I) selon la revendication 1, dans laquelle R¹ a la signification indiquée à la revendication 1 et R² est -NH₂ ou -NHCOOR³, caractérisé en ce que l'on fait réagir des composés de formule générale (II) : 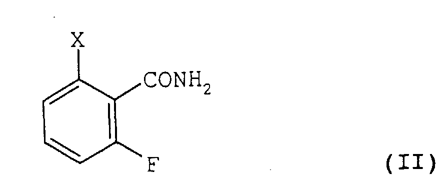 dans laquelle X signifie OR¹, dans un milieu alcalino-aqueux avec du chlore, du brome, de l'hypochlorite de sodium ou de l'hypobromite de sodium en présence d'alcools, à des températures de -15°C à +80°C, au sens d'une dégradation de Hofmann.

6. Procédé selon la revendication 5, caractérisé en ce que la réaction se fait en présence d'un alcool HOR³, où R³ a la même signification que dans la revendication 1.

7. Procédé selon la revendication 6, caractérisé en ce que le composé ainsi obtenu, de formule générale (I') : 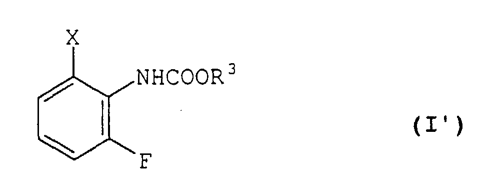 soit est isolé, soit le groupe carbamate et/ou, lorsque X = OR¹, le groupe OR¹ est dissocié par hydrogénolyse ou hydrolyse.

8. Procédé selon l'une au moins des revendications 5 à 7, caractérisé en ce que, pour préparer les composés (II), on part du 2,6-difluorobenzonitrile et on convertit le groupe nitrile d'une manière en soi connue en groupe amide et, dans le cas des composés où X est OR¹, on échange l'atome de F correspondant avant, pendant ou après cette transformation, contre le groupe OR¹.

9. Procédé selon la revendication 8, caractérisé en ce que l'échange de l'atome de fluor contre le groupe benzyloxy se fait en même temps que la conversion du groupe nitrile en groupe amide, de manière à convertir le 2,6-difluorobenzonitrile en présence d'un alcool HOR³, où R³ a la même signification que dans la revendication 1 et représente de préférence un résidu benzyle, en le benzamide correspondant et le cas échéant on dissocie ensuite le groupe OR¹ (R¹ étant ici différent de hydrogène) par hydrogénolyse.

10. Procédé selon la revendication 8, caractérisé en ce que, pour préparer le composé de formule (I) avec R² = CN, on fait réagir le 2,6-difluorobenzonitrile avec un alcool HOR³, où R³ a la même signification que dans la revendication 1, dans un milieu alcalino-aqueux ou dans un solvant aprotique dipolaire en présence d'alcalis.

11. Procédé de préparation des composés de formule (I) selon la revendication 1, dans laquelle R² est NH₂, caractérisé en ce que l'on fait réagir soit le 2,6-difluorobenzonitrile, soit le 2,6-difluorobenzamide en milieu alcalino-aqueux avec des alcools pour obtenir des composés de formule II avec X = OR¹ et on fait réagir ensuite ces derniers, après isolation intermédiaire, mais de préférence toutefois sans isolation intermédiaire, avec du chlore, du brome, de l'hypochlorite de sodium ou de l'hypobromite de sodium, dans un procédé en un seul pot à des températures de -15°C à +80°C en présence d'alcools au sens d'une dégradation de Hofmann, et ensuite on dissocie par hydrolyse ou hydrogénolyse les carbamates éventuellement formés.

12. Procédé selon la revendication 11, caractérisé en ce que l'on utilise comme alcools des phénylméthanols, le résidu alkyle ou le résidu phényle pouvant être substitué par un à trois groupes alcoxy en C₁ à C₄, atomes de fluor, chlore ou brome, groupes nitro, groupes cyano, groupes trifluorométhyle ou groupes alcoxy (en C₁ à C₄)- carbonyle.

13. Procédé selon la revendication 11 ou 12, caractérisé en ce que l'alcool est l'alcool benzylique.

14. Procédé selon l'une au moins des revendications 11 à 13, caractérisé en ce que l'on effectue les conversions en 2-benzyloxy-6-fluorobenzamides dans un milieu alcalino-aqueux ou dans un ou plusieurs solvants aprotiques dipolaires en présence d'alcalis.

15. Procédé selon l'une au moins des revendications 11 à 13, caractérisé en ce que la conversion en composés de formule (II) avec X = OR¹ se fait à des températures de 0°C à 90°C, de préférence de 20°C à 70°C.

16. Procédé selon la revendication 14, caractérisé en ce que la conversion se fait à des températures de 30°C à 150°C, de préférence de 40°C à 100°C.

17. Procédé selon l'une au moins des revendications 11 à 16, caractérisé en ce que, dans le cas des composés obtenus par la dégradation de Hofmann, on dissocie les groupes carbamate qu'ils contiennent et aussi, le cas échéant, les groupes OR¹ par hydrogénolyse et/ou hydrolyse.

18. Procédé selon la revendication 17, caractérisé en ce que la dissociation hydrogénolytique se fait en présence de catalyseurs à base de métaux de transition, notamment des catalyseurs au platine, au nickel ou au palladium.

19. Utilisation des composés de formule (I) selon la revendication 1, notamment des composés dans lesquels
R¹ représente CH₂-phényle et
R² représente -CN, -CONH₂, -NH₂, -NHCOOR³ où R³ est méthyle, éthyle, CH₂-phényle, pour la préparation de composés à cristaux liquides, de produits phytosanitaires ou de produits pharmaceutiques.
